# EUROPEAN PATENT APPLICATION

(11) **EP 2 478 906 A1**
(43) Date of publication of application: **25.07.2012**
(21) Application number: 10817305.5
(22) Date of filing: 17.09.2010
(51) Int. Cl.: A61K 31/5575, A61K 9/08, A61K 31/5377, A61K 47/12, A61K 47/22, A61K 47/34, A61P 27/06

(54) **LATANOPROST-CONTAINING AQUEOUS EYE DROPS AND METHOD FOR INHIBITING ADSORPTION OF LATANOPROST TO RESIN**

(30) Priority: 17.09.2009 JP 2009216182
(71) Applicant: Senju Pharmaceutical Co., Ltd., Osaka-shi, Osaka 541-0046 (JP)
(72) Inventor: NAKAJIMA, Tomoko, Kobe-shi Hyogo 651-2241 (JP); ASAYAMA, Wakiko, Kobe-shi Hyogo 651-2241 (JP); TAJIKA, Tetsuya, Kobe-shi Hyogo 651-2241 (JP)
(74) Representative: Harmsen, Dirk
(86) International application number: PCT/JP2010/066263
(87) International publication number: WO 2011/034192

(57) **Abstract**

The present invention provides an aqueous eye drop containing latanoprost, which is a preparation wherein decrease of an effective concentration of latanoprost due to adsorption to a resin is suppressed and the stability of latanoprost is improved. It is also an object of the present invention to provide a method of suppressing adsorption of latanoprost to a resin.

The present invention provides an aqueous eye drop containing latanoprost, a surfactant, and an aliphatic mono- or di-carboxylic acid having a carbon number of 3 - 10 or a salt thereof. In addition, the present invention provides a method of suppressing adsorption of latanoprost to a resin in an aqueous solution, including adding a surfactant, and an aliphatic mono- or di-carboxylic acid having a carbon number of 3 - 10 or a salt thereof.

## Description

### Technical Field

The present invention relates to an aqueous eye drop containing latanoprost. The present invention also relates to a method of suppressing adsorption of latanoprost to a resin.

### Background Art

At least a part of glaucoma is a group of ophthalmic diseases characterized by progressive optic neuropathy caused by increased intraocular pressure (IOP). In Japan, glaucoma is the second etiological cause after diabetic retinopathy of blindness.

Latanoprost, which is a prostaglandin F_{2α} derivative, has high selectivity to FP receptor in prostaglandin receptors, and provides an effect of lowering the intraocular pressure by increasing the uveoscleral outflow of aqueous humor. Although latanoprost sometimes causes side effects of conjunctival congestion and the like, they are mostly mild and transient, and since instillation once per day is effective and the like, it is widely used as an eye drop for glaucoma treatments.

However, since latanoprost particularly easily adsorbes to resin in an aqueous solvent, when it is filled and preserved in a widely-used resin container, latanoprost problematically adsorbs to the inside of the container to decrease effective concentration thereof in the eye drop. In addition, since latanoprost is easily decomposed by heat in an aqueous solution, latanoprost eye drop requires cold storage. Thus, compliance is considered to decrease in patients with glaucoma or ocular hypertension disease. Moreover, production of ophthalmic compositions such as eye drop and the like often includes a sterilization filtration step using a resin membrane and a filling step using a resin tube and the like. However, since latanoprost easily adsorbes to a filtration membrane, a tube and the like, the decrease of effective concentration of latanoprost has also been a problem during production.

Therefore, various considerations have conventionally been made regarding stabilization of latanoprost in aqueous solutions and suppression of adsorption of latanoprost to resin containers. For example, use of polypropylene containers in which aqueous ophthalmic compositions such as eye solution and the like are to be filled (patent document 1), and resin containers formed of a polymer alloy of poly(ethylene terephthalate) and polyarylate (patent document 2) are disclosed. In addition, a technique for suppressing decomposition of latanoprost in an aqueous solution and adsorption of latanoprost to resin containers by adding a non-ionic surfactant is disclosed (patent documents 1 - 5). As such non-ionic surfactant, polyoxyethylene sorbitan fatty acid ester, polyoxyethylene hydrogenated castor oil, polyoxyethylene-polyoxypropylene glycol, polyethylene glycol stearate, sucrose fatty acid ester and the like are recited (patent document 3). Moreover, suppression of adsorption to containers by polyoxyethylene (20) sorbitan monooleate (polysorbate 80) or polyoxyethylene (60) hydrogenated castor oil is also shown (patent documents 4 and 5). However, the effects of stabilization of latanoprost in aqueous solution, and suppression of adsorption of latanoprost to a resin container in aqueous solvent in these prior arts are not sufficient and further improvement is desired.

As for a non-ionic surfactant tyloxapol, it is disclosed that tyloxapol can be used as a cosolvent of a pharmaceutical composition containing a prostaglandin derivative (patent document 6), and for the preparation of an aqueous ophthalmic oil-in-water type emulsion containing latanoprost (patent document 7).

### [Document List]

### [patent documents]

patent document 1: JP-A-2002-520368
patent document 2: JP-A-2005-60388
patent document 3: WO2008/096804
patent document 4: JP-A-2009-40727
patent document 5: JP-A-2002-161037
patent document 6: JP-A-H10-182465
patent document 7: National Publication of International Patent Application No. 2009-511442

### [SUMMARY OF THE INVENTION]

### Problems to be Solved by the Invention

It is an object of the present invention to provide an aqueous eye drop containing latanoprost, which is a preparation wherein decrease of an effective concentration of latanoprost due to adsorption to a resin is suppressed and the stability of latanoprost (particularly thermal stability) is improved. It is also an object of the present invention to provide a method of suppressing adsorption of latanoprost to a resin.

### Means of Solving the Problems

The present inventors have conducted intensive studies in an attempt to solve the aforementioned problems and found that adsorption of latanoprost to a resin is more remarkably suppressed as compared to conventional methods including addition of a surfactant alone, and the stability of latanoprost in an aqueous solution (particularly thermal stability) is more remarkably improved as compared to the conventional methods, by adding a surfactant, and aliphatic mono- or di-carboxylic acid having a carbon number of 3 - 10 or a salt thereof to an aqueous eye drop containing latanoprost, which resulted in the completion of the present invention.

Accordingly, the present invention relates to the following [1] - [20].
[1] An aqueous eye drop comprising latanoprost, a surfactant, and an aliphatic mono- or di-carboxylic acid having a carbon number of 3 - 10 or a salt thereof.
[2] The aqueous eye drop of the above, further comprising timolol or a salt thereof.
[3] The aqueous eye drop of the above, wherein the aforementioned aliphatic mono- or di-carboxylic acid having a carbon number of 3 - 10 is sorbic acid.
[4] The aqueous eye drop of the above, wherein the aforementioned surfactant is a non-ionic surfactant.
[5] The aqueous eye drop of the above, wherein the aforementioned non-ionic surfactant is selected from the group consisting of polyoxyethylene hydrogenated castor oils, polyoxyethylene fatty acid esters, polyoxyethylene sorbitan fatty acid esters, polyoxyethylene alkyl ethers, polyoxyethylene alkenyl ethers, polyoxyethylene alkylphenyl ethers, polyoxyethylene alkenylphenyl ethers and polyoxyethylene alkynylphenyl ethers.
[6] The aqueous eye drop of the above, wherein the aforementioned non-ionic surfactant is selected from the group consisting of tyloxapol, polyoxyethylene (20) sorbitan oleic acid ester, polyoxyethylene hydrogenated castor oil 40 and polyethylene glycol (40) monostearate.
[7] The aqueous eye drop of the above, wherein the concentration of the aforementioned surfactant is about 0.01 (w/v)% - about 0.1 (w/v)%.
[8] The aqueous eye drop of the above, wherein the concentration of the aforementioned surfactant is about 0.03 (w/v)% - about 0.1 (w/v)%.
[9] The aqueous eye drop of the above, which is filled in a resin container.
[10] The aqueous eye drop of the above, wherein the aforementioned resin container is made from an optionally substituted polyolefin or a polyterephthalic acid ester.
[11] The aqueous eye drop of the above, wherein the aforementioned resin container is a polyethylene container, a polypropylene container, a poly(vinylidene fluoride) container or a poly(ethylene terephthalate) container.
[12] A method of suppressing adsorption of latanoprost to a resin in an aqueous solution, comprising adding a surfactant, and an aliphatic mono- or di-carboxylic acid having a carbon number of 3 - 10 or a salt thereof.
[13] The method of the above, wherein the aforementioned aliphatic mono- or di-carboxylic acid having a carbon number of 3 - 10 is sorbic acid.
[14] The method of the above, wherein the aforementioned surfactant is a non-ionic surfactant.
[15] The method of the above, wherein the aforementioned non-ionic surfactant is selected from the group consisting of polyoxyethylene hydrogenated castor oils, polyoxyethylene fatty acid esters, polyoxyethylene sorbitan fatty acid esters, polyoxyethylene alkyl ethers, polyoxyethylene alkenyl ethers, polyoxyethylene alkylphenyl ethers, polyoxyethylene alkenylphenyl ethers and polyoxyethylene alkynylphenyl ethers.
[16] The method of the above, wherein the aforementioned non-ionic surfactant is selected from the group consisting of tyloxapol, polyoxyethylene (20) sorbitan oleic acid ester, polyoxyethylene hydrogenated castor oil 40 and polyethylene glycol (40) monostearate.
[17] The method of the above, wherein the aforementioned surfactant is contained in about 0.01 (w/v)% - about 0.1 (w/v)%.
[18] The method of the above, wherein the aforementioned surfactant is contained in about 0.03 (w/v)% - about 0.1 (w/v)%.
[19] The method of the above, wherein the aforementioned resin is an optionally substituted polyolefin or a polyterephthalic acid ester.
[20] The method of the above, wherein the aforementioned resin is polyethylene, polypropylene, poly(vinylidene fluoride) or poly(ethylene terephthalate).

In addition, the present invention relates to the following [21] - [51].
[21] An aqueous eye drop comprising (a) latanoprost and (b) tyloxapol.
[22] The aqueous eye drop of the above, further comprising (c) timolol or a salt thereof.
[23] The aqueous eye drop of the above, further comprising (d) a fatty acid having a carbon number of 3 - 10 or a salt thereof.
[24] The aqueous eye drop of the above, further comprising (c) timolol or a salt thereof, and (d) a fatty acid having a carbon number of 3 - 10 or a salt thereof.
[25] The aqueous eye drop of the above, wherein (d) the fatty acid having a carbon number of 3 - 10 is a fatty acid having a carbon number of 3 - 7.
[26] The aqueous eye drop of the above, wherein (d) the fatty acid having a carbon number of 3 - 10 is sorbic acid.
[27] The aqueous eye drop of the above, wherein the concentration of (b) tyloxapol is about 0.01 (w/v)% - about 0.1 (w/v)%.
[28] The aqueous eye drop of the above, wherein the concentration of (b) tyloxapol is about 0.03 (w/v)% - about 0.1 (w/v)%.
[29] The aqueous eye drop of the above, which is filled in a resin container.
[30] The aqueous eye drop of the above, wherein the aforementioned resin container is a container made from an optionally substituted polyolefin (that is, polyalkylene container) or a poly(alkylene terephthalate) container.
[31] The aqueous eye drop of the above, wherein the aforementioned resin container is a polyethylene container, polypropylene container or poly(vinylidene fluoride) container.
[32] A method of suppressing adsorption of latanoprost to a resin in an aqueous solution, comprising adding tyloxapol.
[33] The method of the above, wherein the aforementioned latanoprost is present in an aqueous eye drop.
[34] The method of the above, further comprising timolol or a salt thereof.
[35] The method of the above, further comprising a fatty acid having a carbon number of 3 - 10 or a salt thereof.
[36] The method of the above, further comprising timolol or a salt thereof, and a fatty acid having a carbon number of 3 - 10 or a salt thereof.
[37] The method of the above, wherein the aforementioned fatty acid having a carbon number of 3 - 10 is a fatty acid having a carbon number of 3 - 7.
[38] The method of the above, wherein the aforementioned fatty acid having a carbon number of 3 - 10 is sorbic acid.
[39] The method of the above, comprising adding the aforementioned tyloxapol in about 0.01 (w/v)% - about 0.1 (w/v)%.
[40] The method of the above, comprising adding the aforementioned tyloxapol in about 0.03 (w/v)% - about 0.1 (w/v)%.
[41] The method of the above, wherein the aforementioned resin is optionally substituted polyolefin (that is, polyalkylene) or poly(alkylene terephthalate).
[42] The method of the above, wherein the aforementioned resin is polyethylene, polypropylene or poly(vinylidene fluoride).
[43] A method of improving thermal stability of latanoprost in an aqueous solution, comprising adding tyloxapol.
[44] The method of the above, wherein the aforementioned latanoprost is present in an aqueous eye drop.
[45] The method of the above, further comprising timolol or a salt thereof.
[46] The method of the above, further comprising a fatty acid having a carbon number of 3 - 10 or a salt thereof.
[47] The method of the above, further comprising timolol or a salt thereof, and a fatty acid having a carbon number of 3 - 10 or a salt thereof.
[48] The method of the above, wherein the aforementioned fatty acid having a carbon number of 3 - 10 is a fatty acid having a carbon number of 3 - 7.
[49] The method of the above, wherein the aforementioned fatty acid having a carbon number of 3 - 10 is sorbic acid.
[50] The method of the above, comprising adding the aforementioned tyloxapol in about 0.01 (w/v)% - about 0.1 (w/v)%.
[51] The method of the above, comprising adding the aforementioned tyloxapol in about 0.03 (w/v)% - about 0.1 (w/v)%.

### Effect of the Invention

According to the present invention, an aqueous eye drop showing a remarkably suppressed adsorption of latanoprost to a resin as compared to a conventional preparation containing only a surfactant, and more remarkably improved stability of latanoprost (particularly thermal stability) as compared to such conventional preparation, can be provided by adding a surfactant, and an aliphatic mono- or di-carboxylic acid having a carbon number of 3 - 10 or a salt thereof to an aqueous eye drop containing latanoprost.
According to the method of the present invention, moreover, adsorption of latanoprost to a resin can be more remarkably suppressed by adding a surfactant, and an aliphatic mono- or di-carboxylic acid having a carbon number of 3 - 10 or a salt thereof to an aqueous solution such as an eye drop and the like containing latanoprost, as compared to a conventional method including adding only a surfactant.

### Brief Description of the Drawings

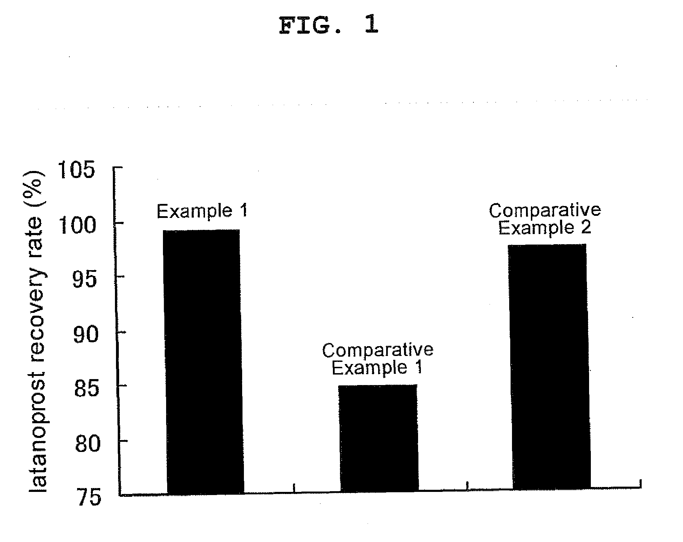

Fig. 1 shows the results of Experimental Example 1.

### [Description of Embodiments]

The definitions of the terms particularly used in the present specification are described in the following.
In the present specification, the "aqueous eye drop" refers to an aqueous liquid for eye drop, and does not include an eye drop in an emulsion form (water-in-oil type, oil-in-water type etc.). Examples of the aqueous eye drop include a monophasic solution such as aqueous solution and the like, an aqueous solution in which an oil-soluble substance is solubilized by micellization, a dispersion or suspension of an insoluble substance in water as a base, and the like.
In the present specification, "latanoprost" refers to a substance represented by a chemical name: (+)-isopropyl-(Z)-7-[(1R,2R,3R,5S)-3,5-dihydroxy-2-[(3R)-3-hydroxy-5-phenylpentyl]cyclopentyl]-5-heptenoate. Latanoprost is a prostaglandin F_{2α} derivative used as a therapeutic drug for glaucoma.
In the present specification, the "surfactant" refers to a substance which separately has hydrophilic moiety and hydrophobicity moiety in a molecule, and has an action of markedly decreasing surface tension by dissolving in a liquid.
In the present specification, the "non-ionic surfactant" refers to a surfactant that is not electrolytically dissociated, and therefore, does not become an ion when dissolved in water.
In the present specification, the "polyoxyethylene hydrogenated castor oils" refer to those having the following structure:

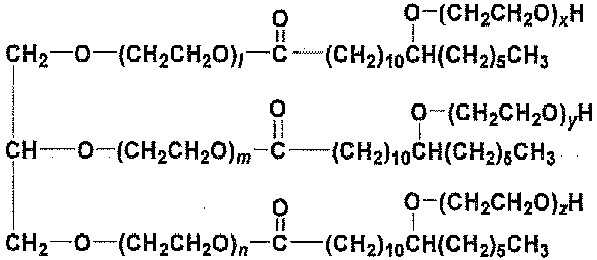

wherein the total of 1, m, n, x, y and z is generally 5 - 100, but is not limited thereto. Specific examples include, but are not limited to, polyoxyethylene hydrogenated castor oil 10, polyoxyethylene hydrogenated castor oil 40, polyoxyethylene hydrogenated castor oil 50, polyoxyethylene hydrogenated castor oil 60, polyoxyethylene hydrogenated castor oil 100 and the like.
In the present specification, the "polyoxyethylene fatty acid esters" refer to those having the following structure: RCOO-(CH₂CH₂O)ₙH wherein R is an alkyl group having a carbon number of generally 12 - 18 or an alkenyl group having a carbon number of generally 12 - 18, said alkyl group and alkenyl group may be straight chain or branched chain, and n is generally 10 - 70, though not limited thereto. Specific examples include, but are not limited to, polyethylene glycol 10 monolaurate, polyethylene glycol 10 monostearate, polyethylene glycol 25 monostearate, polyethylene glycol 40 monostearate, and polyethylene glycol 55 monostearate.
In the present specification, the "polyoxyethylene sorbitan fatty acid esters" refer to those having the following structure:

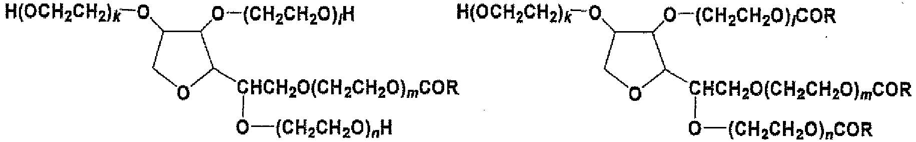

wherein R is an alkyl group having a carbon number of generally 12 - 18, an alkenyl group having a carbon number of generally 12 - 18 or an alkynyl group having a carbon number of generally 12 - 18, said alkyl group, alkenyl group and alkynyl group may be straight chain or branched chain, and the total of k, 1, m and n is generally 20, though not limited thereto. Specific examples include, but are not limited to, polysorbate 80 (polyoxyethylene sorbitan monooleate), polysorbate 60 (polyoxyethylene sorbitan monostearate), polysorbate 40 (polyoxyethylene sorbitan monopalmitate), polyoxyethylene monolaurate, polyethylene sorbitan trioleate, and polysorbate 65 (polyoxyethylene sorbitan tristearate).
In the present specification, the "polyoxyethylene alkyl ethers" and "polyoxyethylene alkenyl ethers" refer to those having the following structure:

R-O-(CH₂CH₂O)ₙH

wherein R is an alkyl group having a carbon number of generally 12 - 18, an alkenyl group having a carbon number of generally 12 - 18, said alkyl group and alkenyl group may be straight chain or branched chain, and n is generally 10 - 50, though not limited thereto.
Specific examples include, but are not limited to, polyoxyethylene lauryl ether, polyoxyethylene stearyl ether, and polyoxyethylene oleyl ether.
In the present specification, the "polyoxyethylene alkylphenyl ethers", "polyoxyethylene alkenylphenyl ethers" and "polyoxyethylene alkynylphenyl ethers" refer to those having the following structure:

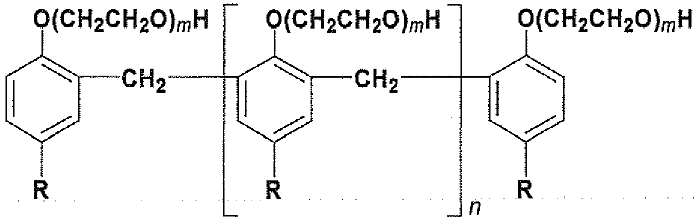

wherein R is an alkyl group having a carbon number of generally 3 - 20 (preferably 8), an alkenyl group having a carbon number of generally 3 - 20 (preferably 8) or an alkynyl group having a carbon number of generally 3 - 20 (preferably 8), said alkyl group, alkenyl group and alkynyl group may be straight chain or branched chain, m is generally 8 - 10, and n is generally 1 - 5, though not limited thereto. Specific examples include, but are not limited to, tyloxapol.
In the present specification, "tyloxapol" refers to a non-ionic surfactant represented by the chemical name: oxyethylated-tert-octylphenolformaldehyde polymer.
In the present specification "timolol" refers to a β blocker represented by the chemical name: 2S-1-[(1,1-dimethylethyl)amino]-3-(4-morpholin-4-yl-1,2,5-thiazol-3-yloxy)propan-2-ol. Timolol suppresses aqueous humor production by an adrenaline β receptor blocking action.
In the present specification, the "fatty acid" refers to a carboxylic acid of hydrocarbon.
In the present specification, the "aliphatic monocarboxylic acid" is a generic term of organic compounds having one carboxyl group in a molecule.
In the present specification, the "aliphatic dicarboxylic acid" is a generic term of organic compounds having 2 carboxyl groups in a molecule.
In the present specification, the "sorbic acid" refers to an unsaturated fatty acid represented by the chemical name: 2,4-hexadienoic acid.
In the present specification, the "resin container" refers to a container formed from a resin as a main material.
In the present specification, the "container made from an optionally substituted polyolefin" refers to a container formed from an optionally substituted polyolefin as a main material. Here, the "optionally substituted polyolefin" refers to, for example, a polyolefin that can be substituted by a substituent such as a halogen atom, a phenyl group and the like.
In the present specification, the "container made from a polyterephthalic acid ester" refers to a container formed from a polyterephthalic acid ester as a main material.
In the present specification, the "polyterephthalic acid ester" refers to a polyester comprised of terephthalic acid and divalent alcohol.

Preferable embodiments of the present invention are explained in the following. It is appreciated that the embodiments provided in the following are for better understanding of the present invention, and the scope of the present invention should not be limited to the following description. Therefore, it is clear that those of ordinary skill in the art can appropriately modify the invention within the scope of the present invention and in consideration of the description of the present specification.

### (aqueous eye drop)

In one aspect, the present invention provides an aqueous eye drop containing latanoprost, a surfactant, and an aliphatic mono- or di-carboxylic acid having a carbon number of 3 - 10 or a salt thereof. Here, examples of the form of the aqueous eye drop include, but are not limited to, a monophasic solution such as aqueous solution and the like, an aqueous solution in which an oil-soluble substance is solubilized by micellization, a dispersion or suspension of an insoluble substance in water as a base and the like. With such constitution, the aqueous eye drop of the present invention can achieve a superior ocular hypotensive action, and a suppressive effect on the adsorption of latanoprost to a resin.

Latanoprost used for the aqueous eye drop of the present invention can be produced according to a known method. The production method of latanoprost is described, for example, in JP-B-2721414 (PHARMACIA & UPJOHN AB), JP-B-3989377 (YONSUN FINE CHEMICALS CO., LTD.) and the like. Latanoprost can also be purchased from SIGMA ALDRICH Co. (product No.: L1167), YONSUN FINE CHEMICALS CO., LTD. and the like.

The content of latanoprost in the aqueous eye drop of the present invention may be, but is not limited to, generally about 0.0005 (w/v)% - about 0.5 (w/v)%, for example, about 0.001 (w/v)% - about 0.1 (w/v)%, about 0.05 (w/v)% - about 0.15 (w/v)%, about 0.075 (w/v)% - about 0.125 (w/v)%, about 0.005 (w/v)% - about 0.015 (w/v)%, about 0.01 (w/v)% - about 0.1 (w/v)%, about 0.0005 (w/v)% - about 0.05 (w/v)%, about 0.0006 (w/v)% - about 0.04 (w/v)%, about 0.0007 (w/v)% - about 0.03 (w/v)%, about 0.0008 (w/v)% - about 0.02 (w/v)%, about 0.0009 (w/v)% - about 0.01 (w/v)%, about 0.001 (w/v)% - about 0.009 (w/v)%, about 0.002 (w/v)% - about 0.008 (w/v)%, about 0.003 (w/v)% - about 0.007 (w/v)%, or about 0.004 (w/v)% - about 0.006 (w/v)%, preferably about 0.001 (w/v)% - about 0.01 (w/v)%, more preferably about 0.0045 (w/v)% - about 0.0055 (w/v)%, about 0.0046 (w/v)% - about 0.0054 (w/v)%, about 0.0047 (w/v)% - about 0.0053 (w/v)%, about 0.0048 (w/v)% - about 0.0052 (w/v)%, about 0.0049 (w/v)% - about 0.0051 (w/v)%, most preferably about 0.0045 (w/v)% - about 0.0054 (w/v)%.
In another embodiment, the content of latanoprost in the aqueous eye drop of the present invention may be about 0.00005 (w/v)% - about 0.005 (w/v)%, about 0.0001 (w/v)% - about 0.001 (w/v)%, about 0.0002 (w/v)% - about 0.0008 (w/v)%, about 0.0003 (w/v)% - about 0.0007 (w/v)%, or about 0.0004 (w/v)% - about 0.0006 (w/v)%.
The amount of latanoprost to be actually administered depends on the individual to be subjected to the treatment, and is preferably an amount optimized to achieve an ocular hypotensive action without accompanying marked side effects. The effective dose can be sufficiently determined by those of ordinary skill in the art.

As the surfactant to be used for the aqueous eye drop of the present invention, non-ionic surfactant is preferable, polyoxyethylene hydrogenated castor oils, polyoxyethylene fatty acid esters, polyoxyethylene sorbitan fatty acid esters, polyoxyethylene alkyl ethers, polyoxyethylene alkenyl ethers, polyoxyethylene alkylphenyl ethers, polyoxyethylene alkenylphenyl ethers and polyoxyethylene alkynylphenyl ethers and the like are more preferable, and tyloxapol, polyoxyethylene (20) sorbitan oleic acid ester, polyoxyethylene hydrogenated castor oil 40 and polyethylene glycol (40) monostearate are particularly preferable, though not limited thereto.
Latanoprost is a hydrophobic compound not easily dissolved in water. Therefore, latanoprost is easily adsorbed to a resin surface by a hydrophobic bond. On the other hand, a surfactant can form a micelle in water, and the micelle can incorporate a hydrophobic compound. When an aqueous solution contains latanoprost, hydrophobic latanoprost is considered to be incorporated in the micelle. Latanoprost incorporated in the micelle does not adsorb to a resin surface, and the surfactant is considered to suppressively act on the adsorption of latanoprost to the resin surface. In the present invention, therefore, a surfactant can be used irrespective of the kind, and any surfactant employed exhibits a suppressive effect on the adsorption of latanoprost to the resin surface.

The content of the surfactant in the aqueous eye drop of the present invention is generally about 0.01 (w/v)% - about 0.1 (w/v)%, preferably about 0.03 (w/v)% - about 0.1 (w/v)%. The surfactant can also be contained in not less than 0.1 (w/v)% as long as eye damages such as staining spot and the like do not occur. The amount of the surfactant can be appropriately changed by those of ordinary skill in the art in consideration of the concentration of latanoprost and the like.

In the aqueous eye drop of the present invention, tyloxapol purchased, for example, from Ruger Chemical Co., Inc (product No.: 1182-1) or SIGMA ALDRICH Co. (product No.: T8761) can be used. Tyloxapol produced according to a known method can also be used.

Examples of the aliphatic mono- or di-carboxylic acid having a carbon number of 3 - 10 to be used for the aqueous eye drop of the present invention include, but are not limited to, linear or branched chain saturated or unsaturated monocarboxylic acid or dicarboxylic acid, for example, propanoic acid (propionic acid), butanoic acid (butyric acid), 2-methylpropanoic acid (isobutyric acid), pentanoic acid (valeric acid), 2,2-dimethylpropanoic acid (pivalic acid), hexanoic acid (caproic acid), heptanoic acid (enanthic acid), octanoic acid (caprylic acid), nonanoic acid (pelargonic acid), decanoic acid (capric acid), propanedioic acid (malonic acid), butanedioic acid (succinic acid), pentanedioic acid (glutaric acid), hexanedioic acid (adipic acid), heptanedioic acid (pimelic acid), octanedioic acid (suberic acid), nonanedioic acid (azelaic acid), decanedioic acid (sebacic acid), (E)-but-2-enoic acid (crotonic acid), (2E,4E)-hexa-2,4-dienoic acid (sorbic acid), (E)-but-2-enedioic acid (fumaric acid), (Z)-but-2-enedioic acid (maleic acid) and the like.
Aliphatic mono- or di-carboxylic acid has a hydrophobic carbon chain moiety and hydrophilic carboxylic acid in a molecule. This is quite similar to the fact that a surfactant has a hydrophilic moiety and a hydrophobic moiety in a molecule. Aliphatic mono- or di-carboxylic acid is considered to dissolve in a liquid and show an action to decrease surface tension like a surfactant, though the level may be different. Since any aliphatic mono- or di-carboxylic acid having such structure can exhibit a suppressive effect on the adsorption of latanoprost to a resin surface, it can be used in the present invention.
Among those, an aliphatic mono- or di-carboxylic acid having a carbon number of 3 - 7 is preferably used. Furthermore, a linear or branched chain saturated or unsaturated monocarboxylic acid or dicarboxylic acid having a carbon number of 4 - 6 is more preferable, and sorbic acid is particularly preferable.

Examples of the salt of aliphatic mono- or di-carboxylic acid having a carbon number of 3 - 10 to be used for the aqueous eye drop of the present invention include alkali metal salts such as sodium salt, potassium salt and the like.
In the aqueous eye drop of the present invention, the content of the aliphatic mono- or di-carboxylic acid having a carbon number of 3 - 10 or a salt thereof varies depending on the kind of the surfactant, but it is generally about 0.01 (w/v)% - about 10 (w/v)%, preferably about 0.02 (w/v)% - about 5 (w/v)%, more preferably about 0.04 (w/v)% - about 2 (w/v)%. When the aqueous eye drop of the present invention is combined with a β blocker (e.g., timolol), those of ordinary skill in the art can appropriately determine the concentration of the aliphatic mono- or di-carboxylic acid in consideration of the intraocular penetration of the β blocker.
Particularly, aliphatic mono- or di-carboxylic acid having a carbon number of 3 - 7 and a salt thereof have been reported to promote intraocular penetration of timolol or a salt thereof (WO99/22715).
When the aqueous eye drop of the present invention contains timolol or a salt thereof, an aliphatic mono- or di-carboxylic acid having a carbon number of 3 - 7 or a salt thereof is preferably used from among the aforementioned aliphatic mono- or di-carboxylic acid having a carbon number of 3 - 10 or a salt thereof. Use of an aliphatic mono- or di-carboxylic acid having a carbon number of 3 - 7 or a salt thereof provides an effect of enhanced aqueous humor penetration of timolol or a salt thereof.
When the aqueous eye drop of the present invention further contains timolol or a salt thereof, the content of the aliphatic mono- or di-carboxylic acid having a carbon number of 3 - 10 (particularly 3 - 7) or a salt thereof is preferably about 0.01 (w/v)% - about 10 (w/v)%, more preferably about 0.04 (w/v)% - about 2 (w/v)%, in consideration of the aqueous humor penetration or intraocular penetration-enhancing effect of the drug.

In another embodiment, the aqueous eye drop of the present invention can contain timolol or a salt thereof. The presence of timolol or a salt thereof can potentiate the suppressive action on the adsorption of latanoprost to a resin.

In the aqueous eye drop of the present invention, timolol widely used as a β blocker can be used. Timolol purchased, for example, from Watanabe-Chemical co., ltd., SAGAMI CHEMICAL INDUSTRY CO., LTD. or SIGMA ALDRICH Co. (product No.: T6394) can be used. As a salt of timolol, any pharmaceutically acceptable salt can be used without any limitation and, for example, acid addition salts such as hydrochloride, sulfate, nitrate, hydrobromide, hydroiodide, phosphate, acetate, maleate, fumarate, citrate, tartrate and the like, and the like can be mentioned. Among these salts, hydrochloride and maleate are preferable, and maleate is particularly preferably used. In the present specification, timolol maleate is also referred to as maleic acid timolol.

When the aqueous eye drop of the present invention contains timolol or a salt thereof, the content thereof can be generally selected from the range where the pharmacological action can be exerted. In one embodiment, when the aqueous eye drop of the present invention contains timolol or a salt thereof, the content thereof is generally about 0.02 (w/v)% - about 3 (w/v)%, preferably about 0.1 (w/v)% - about 2 (w/v)%, though not limited thereto. Those of ordinary skill in the art can appropriately change the content of timolol or a salt thereof according to the symptom of the subject patient.

The aqueous eye drop of the present invention can contain other medicaments, for example, other therapeutic drug for glaucoma, as long as the characteristics of the present invention and the stability of the eye drop are not impaired. Examples of other therapeutic drug for glaucoma include, but are not limited to, dipivefrin hydrochloride (prodrug of epinephrine), befunolol hydrochloride (β blocker), carteolol hydrochloride, betaxolol hydrochloride, nipradilol (drug having α blocking action and β blocking action (αβ blocker)), levobunolol hydrochloride (drug having α₁ blocking action and β blocking action (α₁β blocker)), bunazosin hydrochloride (α₁ blocker), dorzolamide hydrochloride (carbonate dehydratase inhibitor), brinzolamide, isopropyl unoprostone (metabolic prostaglandin drug) and the like. These can be generally added at a concentration within the range employed for ophthalmic compositions (for example, eye drop) or lower than that, and those of ordinary skill in the art can appropriately determine the concentration of these medicaments in consideration of the symptoms of patients.

Moreover, the aqueous eye drop of the present invention can contain additives generally added to eye drops as necessary, as long as the characteristics of the present invention and the stability of the eye drop are not impaired. Examples of such additive include, but are not limited to, isotonicity agents such as sodium chloride, potassium chloride, glycerol, mannitol, sorbitol, boric acid, glucose, propylene glycol and the like; buffering agents such as phosphate buffer, acetate buffer, borate buffer, carbonate buffer, citrate buffer, tris buffer, glutamic acid, ε-aminocaproic acid and the like; preservatives such as benzalkonium chloride, benzethonium chloride, chlorhexidine gluconate, chlorobutanol, benzyl alcohol, sodium dehydroacetate, paraoxybenzoate esters, sodium edetate, boric acid and the like; stabilizers such as sodium bisulfite, sodium thiosulfate, sodium edetate, sodium citrate, ascorbic acid, dibutylhydroxytoluene and the like; water-soluble cellulose derivatives such as methylcellulose, hydroxyethylcellulose, hydroxypropylmethylcellulose, carboxymethylcellulose and the like; thickeners such as sodium chondroitin sulfate, sodium hyaluronate, carboxyvinyl polymer, polyvinyl alcohol, polyvinylpyrrolidone, macrogol and the like; pH adjusters such as hydrochloric acid, sodium hydroxide, phosphoric acid, acetic acid and the like; and the like. While the amount of these additives to be added varies depending on the kind, use and the like of the additive to be added, they only need to be added at a concentration capable of achieving the object of the additive.
The isotonicity agent can be generally added in an amount to make the osmotic pressure ratio about 0.8 - about 1.2. The buffering agent can be added at about 0.01 (w/v)% - about 2 (w/v)%. The stabilizer can be added at about 0.001 (w/v)% - about 1 (w/v)%. The thickener can be added at about 0.001 (w/v)% - about 3 (w/v)%.

The pH of the aqueous eye drop of the present invention can be generally adjusted to about 4.5 - about 8.5, preferably about 5 - about 8, more preferably about 6 - about 7.

The aqueous eye drop of the present invention has a constitution containing latanoprost, a surfactant, and an aliphatic mono- or di- carboxylic acid having a carbon number of 3 - 10 or a salt thereof, whereby adsorption of latanoprost to a resin can be suppressed. Therefore, in a sterilization filtration step using a filter cartridge with a resin filtration membrane and a filling step using a tube, a packing, a valve and the like made of resin, during production of an aqueous eye drop of latanoprost, the adsorption of latanoprost to these resin members can be suppressed.

In consideration of the transport and use of a preparation, an eye drop is conveniently in the form of being filled in a resin container. Even when filled and preserved in a resin container, the aqueous eye drop of the present invention can prevent a decrease of the effective concentration due to the adsorption of latanoprost to the resin container. Moreover, the aqueous eye drop of the present invention can also improve stability to heat. Therefore, the present invention can provide an aqueous eye drop of latanoprost, which is stable even after long-term preservation in a resin container and superior in convenience in terms of transport and use.

When an aqueous eye drop of latanoprost is produced, a sterilization filtration step is often employed as a sterilization method. The prepared aqueous eye drop is generally filled in a container using a tube, a packing, a valve and the like.

Examples of the resin member and resin container used for the production of the aqueous eye drop of the present invention include those formed from the resins shown below as main materials.
Examples of the resin used for a filtration membrane and an outer frame of a filter cartridge in the sterilization filtration step include, but are not limited to, cellulose acetate, aromatic polyamide, polyvinyl alcohol, polyacrylonitrile, polysulfone, polyethylene, polypropylene, poly(vinylidene fluoride), poly(ethylene terephthalate) and the like. Examples of the resin used for a tube, a packing, a valve and the like in the aqueous eye drop filling step include, but are not limited to, silicone resin, polytetrafluoroethylene and the like. Examples of the resin used for a container to be filled with an aqueous eye drop include, but are not limited to, plastic resins such as polyethylene, polypropylene, poly(vinyl chloride), poly(vinylidene chloride), polytetrafluoroethylene, poly(ethylene terephthalate), poly(propylene terephthalate), poly(butylene terephthalate), polystyrene, poly(vinyl acetate), polyurethane, poly(methyl methacrylate), polycarbonate and the like.
In the present invention, for example, polyethylene container (container formed from low density polyethylene (e.g., Tosoh Corporation, 175K), polyethylene container formed by blow-molding low density polyethylene (Hanshin Chemical Industry Co., Ltd., eye drop container)), polypropylene container (e.g., container formed by blow-molding polypropylene (e.g., Japan Polypropylene Corporation, NLB340G), polypropylene container formed by blow-molding polypropylene (Nishiguchi Ampoule Manufacturing Co., Ltd., eye drop container)) and the like can be used. As the resin member and resin container used in the present invention, those commercially available from container companies and the like can be directly used, or can be produced by a known method.
In one embodiment, as the resin member and resin container used for the production of the aqueous eye drop of the present invention, those formed from polyolefin resins such as polyethylene, polypropylene and the like; polyterephthalic acid ester resins such as poly(alkylene terephthalate) such as poly(ethylene terephthalate), poly(propylene terephthalate), poly(butylene terephthalate) and the like, and the like; polyolefin resin substituted by a halogen group, a phenyl group and the like such as poly(vinylidene fluoride), poly(tetrafluoroethylene), polystyrene and the like, and the like as main materials can be preferably used.

The aqueous eye drop of the present invention can be prepared according to the method described in the Japanese Pharmacopoeia, 15th Edition, General rules for preparations, the section of "ophthalmic solution" and the like. For example, the eye drop can be prepared by adding latanoprost, a surfactant, and an aliphatic mono- or di-carboxylic acid having a carbon number of 3 - 10 or a salt thereof and, where necessary, timolol or a salt thereof together with other additives to sterilized purified water, and mixing and dissolving them. Then, where necessary, the dissolved aqueous solution is sterilized by filtration with a filter cartridge and the like made from a resin filtration membrane, and filled in a resin container (e.g., polyethylene container, polypropylene container, poly(vinylidene fluoride) container, poly(ethylene terephthalate) container) using a filling tube and the like.

The aqueous eye drop of the present invention has an effect that adsorption of latanoprost to a resin container can be suppressed even when preserved at room temperature or higher (see Experimental Examples 2, 3 and 6). Therefore, the aqueous eye drop of the present invention can maintain an effective concentration of latanoprost even when it is not preserved in a cold place, and the intraocular pressure-lowering effect of the aqueous eye drop can also be finely maintained. For example, the aqueous eye drop of the present invention can be used at one drop once a day, when it is an aqueous eye drop containing latanoprost at about 0.005 (w/v)%.

### (method of suppressing adsorption of latanoprost to resin)

In another aspect, the present invention provides a method of suppressing adsorption of latanoprost to a resin in an aqueous solution, comprising adding a surfactant and an aliphatic mono- or di-carboxylic acid having a carbon number of 3 - 10 or a salt thereof.

In one embodiment, the aqueous solution containing latanoprost in the adsorption suppression method of the present invention is, for example, an aqueous eye drop containing latanoprost.

In one embodiment, in the adsorption suppression method of the present invention, timolol and/or a salt thereof can be added in addition to a surfactant, and an aliphatic mono- or di-carboxylic acid having a carbon number of 3 - 10 or a salt thereof.

The latanoprost, surfactant, aliphatic mono- or di-carboxylic acid having a carbon number of 3 - 10 or a salt thereof, timolol or a salt thereof, resin and the like to be used here are similar to those described in the aforementioned section, (aqueous eye drop).

The adsorption suppression method of the present invention can finely suppress adsorption of latanoprost to a resin (particularly, polyethylene, polypropylene, poly(vinylidene fluoride), poly(ethylene terephthalate) etc.) in an aqueous solution. As a result, the loss of latanoprost in the production step, which has been the problem, can be successfully suppressed to the minimum. Moreover, the method of the present invention has enabled provision of a stable eye drop that can maintain the effective concentration of latanoprost for a long time even when it is preserved in a resin container.

### (method of improving thermal stability of latanoprost)

In another aspect, the present invention provides a method of improving thermal stability of latanoprost in an aqueous solution, comprising adding a surfactant, and an aliphatic mono- or di-carboxylic acid having a carbon number of 3 - 10 or a salt thereof.

In one embodiment, the aqueous solution containing latanoprost in the thermal stability improving method of the present invention is, for example, an aqueous eye drop containing latanoprost.

In one embodiment, in the thermal stability improving method of the present invention, timolol or a salt thereof can be added in addition to a surfactant, and an aliphatic mono- or di-carboxylic acid having a carbon number of 3 - 10 or a salt thereof.

As the latanoprost, surfactant, aliphatic mono- or di-carboxylic acid having a carbon number of 3 - 10 or a salt thereof, timolol or a salt thereof, resin and the like, to be used here, those similar to the ones described in the aforementioned section, (aqueous eye drop) can be used.

Due to the unstability to heat, conventional latanoprost eye drops require preservation in a cold place. The method of the present invention can improve thermal stability of latanoprost in an aqueous solution, thus making it possible to provide an aqueous eye drop of latanoprost which is stable even when preserved at room temperature for a long time. Such aqueous eye drop of latanoprost is provided for the first time by the present invention.

The present invention also relates to an aqueous eye drop comprising (a) latanoprost and (b) tyloxapol (hereinafter to be referred to as the aqueous eye drop A of the present invention), and a method of suppressing adsorption of latanoprost to a resin in an aqueous solution, comprising adding tyloxapol (hereinafter to be referred to as the method A of the present invention). By combining latanoprost and tyloxapol, the adsorption of latanoprost to a resin can be finely suppressed, which in turn prevents loss of latanoprost due to the adsorption to a resin in the production stage. Moreover, the method A of the present invention can provide a stable aqueous eye drop of latanoprost, which is free of a decrease of the effective concentration during preservation.
The aqueous eye drop A of the present invention can contain (c) timolol or a salt thereof, and (d) fatty acid having a carbon number of 3 - 10 or a salt thereof. As the latanoprost, tyloxapol, timolol, resin and the like, those similar to the ones described in the aforementioned section (aqueous eye drop) can be used. As the fatty acid having a carbon number of 3 - 10, one similar to the aliphatic mono- or di-carboxylic acid having a carbon number of 3 - 10 described in the aforementioned section (aqueous eye drop) can be used. Examples

The present invention is explained in detail in the following by referring to Examples, which are not to be construed as limitative.

### [Example 1] Aqueous eye drop

The formulation of the aqueous eye drop of Example 1 is shown in Table 1 together with the formulations of the aqueous eye drops of Comparative Examples 1 and 2. For the preparation thereof, firstly, (1) in Table 1 was added to (8) (10% benzalkonium chloride solution), and the mixture was dissolved by stirring at about 50°C to give a latanoprost stock solution. (2) - (7) and an appropriate amount of (11) were placed in a different container, and the mixture was dissolved by stirring to give a base stock solution. The entire amount of the base stock solution was added to the latanoprost stock solution, and the mixture was thoroughly stirred. (9) and (10) were added to adjust the mixture to pH 6.7, and (11) was added to the total amount of 1 L.

**Table 1**

| component | | content (w/v%) | | |
|---|---|---|---|---|
| | | Example 1 | Comparative Example 1 | Comparative Example 2 |
| (1) | latanoprost | 0.005 | 0.005 | 0.005 |
| (2) | tyloxapol | 0.06 | - | 0.06 |
| (3) | timolol maleate | 0.68 | - | - |
| (4) | potassium sorbate | 0.47 | - | - |
| (5) | sodium chloride | 0.46 | 0.75 | 0.75 |
| (6) | sodium dihydrogen phosphate | 0.05 | 0.05 | 0.05 |
| (7) | sodium hydrogen phosphate hydrate | 0.15 | 0.15 | 0.15 |
| (8) | benzalkonium chloride | 0.02 | 0.02 | 0.02 |
| (9) | hydrochloric acid | q.s. | q.s. | q.s. |
| (10) | sodium hydroxide | q.s. | q.s. | q.s. |
| (11) | sterile purified water | q.s. | q.s. | q.s. |

### [Experimental Example 1]

Each 50 mL of the aqueous eye drops prepared in Example 1 and Comparative Examples 1 and 2 was filtered through a poly(vinylidene fluoride) resin membrane (Durapore membrane filter GVWP04700, manufactured by Nihon Millipore K.K.). The amount of the latanoprost contained in the filtrate was quantified, and a recovery rate (%) relative to the content of latanoprost in each aqueous eye drop before filtration as 100% was calculated. The results are shown in Fig. 1. Latanoprost was quantified by high performance liquid chromatography (HPLC) under the following HPLC measurement condition I.

### <HPLC measurement condition I>

Measurement sample preparation: Each latanoprost eye solution (2 mL) was precisely measured, dilution 1 (pH 2.0 trifluoroacetic acid solution/acetonitrile (1:2)) was added to make the total precisely 5 mL and the mixture was used as a sample solution. Separately, a latanoprost standard product (about 0.1 g) was precisely measured, dissolved by adding acetonitrile to make the total precisely 100 mL and the mixture was used as a standard stock solution. The standard stock solution (4 mL) was precisely measured, and acetonitrile was added to make the total precisely 20 mL. This solution (2 mL) was precisely measured, dilution 2 (pH 3.5 trifluoroacetic acid solution/acetonitrile (2:1)) was added to make the total precisely 20 mL and the mixture was used as a standard solution. The sample solution and standard solution (each 100 uL) were subjected to a test by liquid chromatography under the following conditions, and the peak areas Atl and Asl of latanoprost were measured by automatic integration.
- amount of latanoprost in sample solution (% of labeled amount) =measured weight (mg) of latanoprost standard productxpurity of latanoprost standard product×Atl/Asl
- Atl: latanoprost peak area of sample solution
- Asl: latanoprost peak area of standard solution detector: ultraviolet absorption spectrophotometer

### (measurement wavelength: 210 nm)

column: stainless tube (inner diameter 4.6 mm, length 250 mm) filled with octadecylsilylated silica gel (5 µm) for liquid chromatography (YMC-PACK ODS-A, AA12S05-2546WT, 4.6 mm×250 mm, 5 µm, manufactured by YMC Co., Ltd.)
column temperature: fixed temperature near 40°C
mobile phase A: pH 3.5 trifluoroacetic acid
solution/acetonitrile/methanol mixed solution (13:6:6)
mobile phase B: acetonitrile/methanol mixed solution (1:1) feed of mobile phase: liner gradient controlled by changing mixing ratio of mobile phase A and mobile phase B as shown in Table 2.

**Table 2**

| time (min) after injection | mobile phase A (%) | mobile phase B (%) |
|---|---|---|
| 0 - 110 | 100 | 0 |
| 110 - 115 | 100 → 25 | 0 → 75 |
| 115 - 120 | 25 | 75 |
| 120 - 121 | 25 → 100 | 75 → 0 |
| 121 - 145 | 100 | 0 |

| | | |
|---|---|---|
| flow: controlled such that retention time of latanoprost was about 100 min | | |

The latanoprost recovery rate of the aqueous eye drop of Example 1 was 99.1%, which shows that latanoprost is hardly adsorbed to a resin membrane (Fig. 1). On the other hand, the latanoprost recovery rate of the aqueous eye drop of Comparative Example 1 was 84.7%, from which it is considered that about 15% of latanoprost was adsorbed to the resin membrane.

### [Experimental Example 2]

Each 5 mL of the aqueous eye drops of Example 1 and Comparative Examples 1 and 2 was filled in two glass ampoules (DAIWA SPECIAL GLASS Co., Ltd., 5 mL colorless powder glass ampoule) and two polyethylene containers (manufactured by Hanshin Chemical Industry Co., Ltd.) obtained by blow-molding low density polyethylene, and they were used as samples. Each sample was preserved at 40°C and 60°C for 2 weeks, latanoprost was quantified by HPLC (HPLC measurement condition I) in the same manner as in the above-mentioned Experimental Example 1, and the ratio (%) relative to the content of each aqueous eye drop before preservation was calculated and is shown in Table 3.

**Table 3**

| preservation conditions | | Latanoprost content (%)* | | |
|---|---|---|---|---|
| container | temperature | Example 1 | Comparative Example 1 | Comparative Example 2 |
| glass ampoule | 40°C | 98.6 | 96.7 | 97.9 |
| | 60°C | 96.8 | 93.6 | 94.4 |
| polyethylene container | 40°C | 97.7 | 94.1 | 96.6 |
| | 60°C | 92.7 | 87.6 | 90.4 |

| | | | | |
|---|---|---|---|---|
| *; ratio (%) relative to latanoprost content of each aqueous eye drop before preservation | | | | |

Any samples of the aqueous eye drop of Example 1 filled in glass ampoules and polyethylene containers showed less decrease of the latanoprost content as compared to the samples of Comparative Example 1 (Table 3). Particularly, when filled in the polyethylene containers, the aqueous eye drop of Example 1 showed remarkable suppression of the adsorption of latanoprost as compared to the aqueous eye drop of Comparative Example 1. In addition, the aqueous eye drop of Example 1 was also stable to heat as compared to the aqueous eye drop of Comparative Example 1.

### [Experimental Example 3]

Each 5 mL of the aqueous eye drops of Example 1 and Comparative Examples 1 and 2 were filled in six polyethylene containers obtained by blow-molding low density polyethylene (manufactured by Hanshin Chemical Industry Co., Ltd.), and six polypropylene containers obtained by blow-molding polypropylene (manufactured by Nishiguchi Ampoule Manufacturing Co., Ltd.), and they were used as samples. Each sample was preserved at 25°C, 40°C and 60°C for 8 days and 16 days, latanoprost was quantified by HPLC (HPLC measurement condition I) in the same manner as in the above-mentioned Experimental Example 1, and the ratio (%) relative to the content of each aqueous eye drop before preservation was calculated and is shown in Table 4.

**[Table 4]**

| preservation conditions | | | Latanoprost content (%)* | | |
|---|---|---|---|---|---|
| container | temperature | period | Example 1 | Comparative Example 1 | Comparative Example 2 |
| polyethylene container | 25°C | 8 days | 99.7 | 100.0 | 99.2 |
| | 40°C | | 98.7 | 97.8 | 99.1 |
| | 60°C | | 93.8 | 86.0 | 91.7 |
| | 25°C | 16 days | 99.6 | 99.5 | 99.9 |
| | 40°C | | 97.6 | 96.2 | 97.3 |
| | 60°C | | 91.5 | 83.7 | 90.2 |
| polypropylene container | 25°C | 8 days | 99.1 | 100.0 | 100.6 |
| | 40°C | | 98.8 | 98.1 | 99.6 |
| | 60°C | | 95.0 | 89.8 | 96.1 |
| | 25°C | 16 days | 98.5 | 99.0 | 100.5 |
| | 40°C | | 97.0 | 97.1 | 99.0 |
| | 60°C | | 93.0 | 81.8 | 92.0 |

| | | | | | |
|---|---|---|---|---|---|
| *; ratio (%) relative to latanoprost content of each aqueous eye drop before preservation | | | | | |

When filled in any of the polyethylene containers and polypropylene containers, the content of latanoprost decreased with increasing preservation temperature (Table 4). However, the aqueous eye drop of Example 1 showed suppression of the decrease of the latanoprost content with increasing preservation temperature, as compared to the aqueous eye drop of Comparative Example 1. In addition, when preserved at 25°C, no significant difference in the latanoprost content was observed between respective samples, irrespective of the containers used. However, when preserved at 60°C, the aqueous eye drop of Example 1 showed marked suppression of the decrease of the latanoprost content as compared to the aqueous eye drop of Comparative Example 1, irrespective of the containers used.

### [Examples 2 - 6] Aqueous eye drop

The formulations of the aqueous eye drops of Examples 2-6 are shown in Table 5 together with the formulation of the aqueous eye drop of Comparative Example 3. In Table 5, the value of each component shows a content ((w/v)%). In addition, "q.s." for hydrochloric acid and sodium hydroxide shows an amount necessary for adjusting the aqueous eye drop to pH 6.7, and that for purified water shows an amount necessary to make the total amount 100 (w/v)%. These aqueous eye drops were prepared in the same manner as in Example 1.

### [Experimental Example 4]

The aqueous eye drops of Examples 2-6 and Comparative Example 3 were subjected to a resin membrane adsorption test in the same manner as in Experimental Example 1. The results are also shown in Table 5.

**[Table 5]**

| component | | **Ex. 2** | **Ex. 3** | **Ex. 4** | **Ex. 5** | **Ex. 6** | **Comp. Ex. 3** |
|---|---|---|---|---|---|---|---|
| (1) | latanoprost | **0.005** | **0.005** | **0.005** | **0.005** | **0.005** | **0.005** |
| (2) | timolol maleate | **0.68** | **0.68** | **0.68** | **0.68** | - | **-** |
| (3) | potassium sorbate | **0.47** | **0.47** | **0.47** | **0.47** | **0.47** | - |
| (4) | tyloxapol | **0.01** | **0.03** | **0.05** | **0.1** | **0.05** | - |
| (5) | sodium chloride sodium | 0.75 | **0.75** | **0.75** | **0.75** | **0.75** | **0.75** |
| (6) | dihydrogen phosphate | **0.05** | **0.05** | **0.05** | **0.05** | **0.05** | **0.05** |
| (7) | sodium hydrogen phosphate hydrate | **0.15** | **0.15** | **0.15** | **0.15** | **0.15** | **0.15** |
| (8) | benzalkonium chloride | 0.02 | **0.02** | **0.02** | **0.02** | **0.02** | **0.02** |
| (9) | hydrochloric acid | q.s. | q.s. | q.s. | q.s. | q.s. | q.s. |
| (10) | sodium hydroxide | q.s. | q.s. | q.s. | q.s. | q.s. | q.s. |
| (11) | purified water | q.s. | q.s. | q.s. | q.s. | q.s. | q.s. |
| pH | | 6.7 | 6.7 | 6.7 | 6.7 | 6.7 | 6.7 |
| Latanoprost recovery rate (%)* | | 85.9 | 94.5 | 96.4 | 97.7 | 96.2 | 82.7 |

| | | | | | | | |
|---|---|---|---|---|---|---|---|
| *; ratio (%) relative to content in each aqueous eye drop before filtration | | | | | | | |

The aqueous eye drops of Examples 2-6 showed higher latanoprost recovery rates as compared to the aqueous eye drop of Comparative Example 3 (Table 5). Particularly, the aqueous eye drops of Examples 3-6 containing tyloxapol in not less than 0.03 (w/v)% showed a markedly high latanoprost recovery rate. From these results, it has been clarified that the aqueous eye drop of the present invention containing tyloxapol in at least 0.01 (w/v)% can suppress adsorption of latanoprost to a resin.

### [Examples 7 - 11] Aqueous eye drop

In these Examples, formulations free of benzalkonium chloride that interacts with sorbic acid were used to accurately evaluate the effect of surfactant and sorbic acid to suppress adsorption of latanoprost to a resin.
The formulations of the aqueous eye drops of Examples 7-11 and Comparative Examples 10-13 are shown in Table 6. In Table 7, the value of each component shows a content (w/v%).
The aqueous eye drops of Examples 7-11 and Comparative Examples 10-13 were prepared by, in Table 6, adding (1)-(10) to (14), stirring the mixture at about 80°C for dissolution, and adjusting to pH 6.7 with (12) and (13), and adding (14) to the total amount of 200 mL.

### [Experimental Example 5]

Each 50 mL of the aqueous eye drops prepared in Examples 7-11 and Comparative Examples 10-13 was filtered through a poly(vinylidene fluoride) resin membrane (Durapore membrane filter GVWP04700, manufactured by Nihon Millipore K.K.). The amount of the latanoprost contained in the filtrate was quantified, and a recovery rate (%) relative to the content of latanoprost in each aqueous eye drop before filtration as 100% was calculated. The results are shown in Fig. 6. Latanoprost was quantified by high performance liquid chromatography according to HPLC measurement condition I.

**[Table 6]**

| component | | content (w/v%) | | | | | | | | |
|---|---|---|---|---|---|---|---|---|---|---|
| | | **Comp. Ex. 10** | **Comp. Ex. 11** | **Ex. 7** | **Ex. 8** | **Ex. 9** | **Ex. 10** | **Comp. Ex. 12** | **Comp. Ex. 13** | **Ex. 11** |
| (1) | latanoprost | **0.005** | **0.005** | **0.005** | **0.005** | **0.005** | **0.005** | **0.005** | **0.005** | **0.005** |
| (2) | timolol maleate | - | **0.68** | **0.68** | **0.68** | **0.68** | **0.68** | - | - | - |
| (3) | potassium sorbate | - | **0.47** | **0.47** | **0.47** | **0.47** | **0.47** | **0.47** | - | **0.47** |
| (4) | tyloxapol | - | - | **0.06** | - | - | - | - | **0.06** | **0.06** |
| (5) | polyoxyethylene (20) sorbitan monooleate | - | - | - | **0.06** | - | - | - | - | - |
| (6) | polyoxyethylene hardened castor oil 40 | - | - | - | - | **0.06** | - | - | - | - |
| (7) | polyethylene glycol (40) monostearate | - | - | - | - | - | **0.06** | - | - | - |
| (8) | sodium chloride | **0.46** | **0.46** | **0.46** | **0.46** | **0.46** | **0.46** | **0.46** | **0.46** | **0.46** |
| (9) | sodium dihydrogen phosphate | **0.05** | **0.05** | **0.05** | **0.05** | **0.05** | **0.05** | **0.05** | **0.05** | **0.05** |
| (10) | sodium hydrogen phosphate hydrate | **0.15** | **0.15** | **0.15** | **0.15** | **0.15** | **0.15** | **0.15** | **0.15** | **0.15** |
| (11) | benzalkonium chloride | - | - | - | - | - | - | - | - | - |
| (12) | hydrochloric acid | q.s. | q.s. | q.s. | q.s. | q.s. | q.s. | q.s. | q.s. | q.s. |
| (13) | sodium hydroxide | q.s. | q.s. | q.s. | q.s. | q.s. | q.s. | q.s. | q.s. | q.s. |
| (14) | purified water | q.s. | q.s. | q.s. | q.s. | q.s. | q.s. | q.s. | q.s. | q.s. |
| pH | | 6.7 | 6.7 | 6.7 | 6.7 | 6.7 | 6.7 | 6.7 | 6.7 | 6.7 |
| Latanoprost recovery rate (ratio to Comp. Ex. 10) | | **1.00** | **1.02** | **1.12** | **1.14** | **1.12** | **1.13** | **1.02** | **1.10** | **1.11** |

In Table 6, the recovery rate of each group shows a calculated value in ratio relative to the recovery rate (%) of Comparative Example 10 as 1.

The aqueous eye drops of Examples 7-10 showed higher latanoprost recovery rates as compared to the aqueous eye drop without a surfactant of Comparative Example 11 (Table 6). In Example 11, the latanoprost recovery rate was higher as compared to the aqueous eye drop without a surfactant of Comparative Example 12 and the aqueous eye drop without potassium sorbate of Comparative Example 13 (Table 6). From these results, it has been clarified that the aqueous eye drop of the present invention containing a surfactant such as tyloxapol and the like, and aliphatic mono- or di-carboxylic acid or a salt thereof specified by the present invention such as calcium sorbate and the like markedly suppresses adsorption of latanoprost to a resin.

### [Examples 12 - 15] Aqueous eye drop

The formulations of the aqueous eye drops of Examples 12 - 15 and Comparative Example 14 are shown in Table 8. In Table 8, the value of each component shows a content (w/v%). For the preparation thereof, firstly, (1) in Table 8 was added to (11) (10% benzalkonium chloride solution), and the mixture was dissolved by stirring at about 50°C to give a latanoprost stock solution. (2) - (10) and an appropriate amount of (14) were placed in a different container, and the mixture was dissolved by stirring to give a base stock solution. The entire amount of the base stock solution was added to the latanoprost stock solution, and the mixture was thoroughly stirred. (12) and (13) were added to adjust the mixture to pH 6.7, and (14) was added to the total amount of 1 L.

### [Experimental Example 6]

Each 5 mL of the aqueous eye drops of Examples 12-15 and Comparative Example 14 were filled in polyethylene containers obtained by blow-molding low density polyethylene (manufactured by Hanshin Chemical Industry Co., Ltd.), and polypropylene containers obtained by blow-molding polypropylene (manufactured by Nishiguchi Ampoule Manufacturing Co., Ltd.), and they were used as samples. Each sample was preserved at 60°C for 4 weeks, and latanoprost in the sample was quantified by high performance liquid chromatography (HPLC). The ratio (%) relative to the content of each aqueous eye drop before preservation was calculated and is shown in Table 8. The conditions (HPLC measurement condition II) of the high performance liquid chromatography are shown below.

### <HPLC measurement condition II>

Measurement sample preparation: Each latanoprost eye drop (2 mL) was precisely measured, dilution 3 (0.2% acetic acid solution/acetonitrile (48:52)) was added to make the total precisely 5 mL and the mixture was used as a sample solution. Separately, a latanoprost standard product (about 0.03 g) was precisely measured, dissolved by adding acetonitrile to make the total precisely 30 mL and the mixture was used as a standard stock solution. The standard stock solution (4 mL) was precisely measured, and acetonitrile was added to make the total precisely 20 mL. This solution (2 mL) was precisely measured, dilution 3 was added to make the total precisely 20 mL and the mixture was used as a standard solution. The sample solution and standard solution (each 50 µL) were subjected to a test by liquid chromatography under the following conditions, and the peak areas Atl and Asl of latanoprost were measured by automatic integration.
- amount of latanoprost in sample solution (% of labeled amount) =measured weight (mg) of latanoprost standard product×100/30×purity of latanoprost standard product×Atl/Asl
- Atl: latanoprost peak area of sample solution
- Asl: latanoprost peak area of standard solution detector: ultraviolet absorption spectrophotometer (measurement wavelength: 210 nm)

column: stainless tube (inner diameter 4.6 mm, length 250 mm) filled with octadecylsilylated silica gel (5 µm) for liquid chromatography
(TSK-GEL, ODS-80TS QA, 4.6 mm×250 mm, 5 µm, manufactured by Tosoh Corporation)
column temperature: fixed temperature near 40°C
mobile phase A: 0.2% acetic acid solution/acetonitrile (48:52)
mobile phase B: 100% acetonitrile
feed of mobile phase: liner gradient controlled by changing mixing ratio of mobile phase A and mobile phase B as shown in Table 8.

**[Table 7]**

| time after injection (min) | mobile phase A (%) | mobile phase B (%) |
|---|---|---|
| 0 - 17 | 100 | 0 |
| 17 - 22 | 100 → 0 | 0 → 100 |
| 22 - 27 | 0 | 100 |
| 27 - 29 | 0 → 100 | 100 → 0 |
| 29 - 50 | 100 | 0 |

| | | |
|---|---|---|
| flow: controlled such that retention time of latanoprost was about 14 min | | |

**[Table 8]**

| component | | | content (w/v%) | | | | |
|---|---|---|---|---|---|---|---|
| | | | **Ex. 12** | **Ex. 13** | **Ex. 14** | **Ex. 15** | **Comp. Ex. 14** |
| (1) | latanoprost | | **0.005** | **0.005** | **0.005** | **0.005** | **0.005** |
| (2) | timolol maleate | | **0.68** | **0.68** | **0.68** | **0.68** | - |
| (3) | potassium sorbate | | **0.47** | **0.47** | **0.47** | **0.47** | - |
| (4) | tyloxapol | | **0.05** | - | - | - | - |
| (5) | polyoxyethylene hardened castor oil 40 | | - | **0.05** | - | - | - |
| (6) | polyethylene glycol (25) monostearate | | - | - | **0.05** | - | - |
| (7) | polyethylene glycol (40) monostearate | | **-** | - | - | **0.05** | - |
| (8) | sodium chloride | | **0.75** | **0.75** | **0.75** | **0.75** | **0.75** |
| (9) | sodium dihydrogen phosphate | | **0.05** | **0.05** | **0.05** | **0.05** | **0.05** |
| (10) | sodium hydrogen phosphate hydrate | | **0.15** | **0.15** | **0.15** | **0.15** | **0.15** |
| (11) | benzalkonium chloride | | **0.02** | **0.02** | **0.02** | **0.02** | **0.02** |
| (12) | hydrochloric acid | | q.s. | q.s. | q.s. | q.s. | q.s. |
| (13) | sodium hydroxide | | q.s. | q.s. | q.s. | q.s. | q.s. |
| (14) | purified water | | q. s. | q.s. | q.s. | q.s. | q.s. |
| pH | | | 6.7 | 6.7 | 6.7 | 6.7 | 6.7 |
| polyethylene container | | 60°C/4 weeks | **89.6** | **90.6** | **91.0** | **89.5** | **78.9** |
| polypropylene container | | 60°C/4 weeks | **93.0** | **93.3** | **91.4** | **92.3** | **80.2** |

The aqueous eye drops of Examples 12-15 showed higher latanoprost recovery rates as compared to the aqueous eye drop without a surfactant and potassium sorbate of Comparative Example 14 (Table 8). From these results, it has been clarified that the aqueous eye drop of the present invention containing a surfactant such as tyloxapol and the like, and aliphatic mono- or di-carboxylic acid or a salt thereof specified by the present invention such as potassium sorbate and the like markedly suppresses adsorption of latanoprost to a resin. In addition, the aqueous eye drops of Examples 12-15 were also stable to heat as compared to the aqueous eye drop of Comparative Example 14.

### [Experimental Example 7] Penetration test of timolol to aqueous humor

WO99/22715 describes that when an eye drop containing timolol maleate and sorbic acid was instilled to the eyes of a rabbit, the penetration of timolol to aqueous humor is enhanced as compared to an eye drop of timolol maleate.
Example 1 and Comparative Example 15 (Table 9) were administered to the both eyes of a rabbit, and the concentration of the timolol in the aqueous humor was measured at 0.5 hr which is the maximum concentration time point (Tₘₐₓ). The concentrations (mean±standard deviation, n=10) of timolol in the aqueous humor at 0.5 hr after the administration of Example 1 and Comparative Example 15 were 3491±1448 ng/mL and 3239±1675 ng/mL, respectively, and no statistically significant difference was observed.
From the above, it has been confirmed that, the concentrations of timolol in the aqueous humor at Tₘₐₓ after administration of Example 1 and Comparative Example 15 are of the same level, and addition of latanoprost does not influence the enhanced penetration of timolol by sorbic acid.

**[Table 9]**

| | Ex. 1 | Comp. Ex. 15 |
|---|---|---|
| latanoprost | 0.005 g | ------- |
| timolol maleate | 0.68 g | 0.68 g |
| potassium sorbate | 0.47 g | 0.47 g |
| tyloxapol | 0.06 g | ------- |
| sodium chloride | 0.46 g | 0.55 g |
| sodium dihydrogen phosphate | 0.05 g | 0.102 g |
| sodium hydrogen phosphate hydrate | 0.15 g | ------- |
| benzalkonium chloride | 0.02 g | 0.005 g |
| hydrochloric acid | q.s. | q.s. |
| sodium hydroxide | q.s. | q,s. |
| purified water | q.s. | q.s. |
| total amount | 100 mL | 100 mL |
| pH | 6.7 | 6.7 |

As shown above, while the present invention has been described with reference to the preferable embodiments of the present invention, the present invention should not be interpreted as being limited to the embodiments. It is appreciated that the scope of the present invention should be interpreted based only on the Claims. It is appreciated that those of ordinary skill in the art can practice the scope equivalent to the description of specific and preferable embodiments of the present invention, based on the description of the present invention and technical common knowledge. It is appreciated that the patent, patent application and document cited in the present specification should be quoted as reference to the present specification, the same way the contents themselves are specifically described in the present specification.

### Industrial Applicability

The present invention is usefulness for suppression of adsorption of latanoprost to a resin. Therefore, the present invention provides a production method and a preservation method of an aqueous eye drop that suppresses the loss of latanoprost to the minimum. From the foregoing, the present invention provides an aqueous eye drop of latanoprost, which can be preserved stably at room temperature for a long time.

This application is based on a patent application No. 2009-216182 filed in Japan, the contents of which are incorporated in full herein.

## Claims

1. An aqueous eye drop comprising latanoprost, a surfactant, and an aliphatic mono- or di-carboxylic acid having a carbon number of 3 - 10 or a salt thereof.

2. The aqueous eye drop according to claim 1, further comprising timolol or a salt thereof.

3. The aqueous eye drop according to claim 1 or 2, wherein the aforementioned aliphatic mono- or di-carboxylic acid having a carbon number of 3 - 10 is sorbic acid.

4. The aqueous eye drop according to any of claims 1 to 3, wherein the aforementioned surfactant is a non-ionic surfactant.

5. The aqueous eye drop according to claim 4, wherein the aforementioned non-ionic surfactant is selected from the group consisting of polyoxyethylene hydrogenated castor oils, polyoxyethylene fatty acid esters, polyoxyethylene sorbitan fatty acid esters, polyoxyethylene alkyl ethers, polyoxyethylene alkenyl ethers, polyoxyethylene alkylphenyl ethers, polyoxyethylene alkenylphenyl ethers and polyoxyethylene alkynylphenyl ethers.

6. The aqueous eye drop according to claim 4, wherein the aforementioned non-ionic surfactant is selected from the group consisting of tyloxapol, polyoxyethylene (20) sorbitan oleic acid ester, polyoxyethylene hydrogenated castor oil 40 and polyethylene glycol (40) monostearate.

7. The aqueous eye drop according to any of claims 1 to 6, wherein the concentration of the aforementioned surfactant is about 0.01 (w/v)% - about 0.1 (w/v)%.

8. The aqueous eye drop according to any of claims 1 to 6, wherein the concentration of the aforementioned surfactant is about 0.03 (w/v)% - about 0.1 (w/v)%.

9. The aqueous eye drop according to any of claims 1 to 8, which is filled in a resin container.

10. The aqueous eye drop according to claim 9, wherein the aforementioned resin container is made from an optionally substituted polyolefin or a polyterephthalic acid ester.

11. The aqueous eye drop according to claim 9, wherein the aforementioned resin container is a polyethylene container, a polypropylene container, a poly(vinylidene fluoride) container or a poly(ethylene terephthalate) container.

12. A method of suppressing adsorption of latanoprost to a resin in an aqueous solution, comprising adding a surfactant, and an aliphatic mono- or di-carboxylic acid having a carbon number of 3 - 10 or a salt thereof.

13. The method according to claim 12, wherein the aforementioned aliphatic mono- or di-carboxylic acid having a carbon number of 3 - 10 is sorbic acid.

14. The method according to claim 12 or 13, wherein the aforementioned surfactant is a non-ionic surfactant.

15. The method according to claim 14, wherein the aforementioned non-ionic surfactant is selected from the group consisting of polyoxyethylene hydrogenated castor oils, polyoxyethylene fatty acid esters, polyoxyethylene sorbitan fatty acid esters, polyoxyethylene alkyl ethers, polyoxyethylene alkenyl ethers, polyoxyethylene alkylphenyl ethers, polyoxyethylene alkenylphenyl ethers and polyoxyethylene alkynylphenyl ethers.

16. The method according to claim 14, wherein the aforementioned non-ionic surfactant is selected from the group consisting of tyloxapol, polyoxyethylene (20) sorbitan oleic acid ester, polyoxyethylene hydrogenated castor oil 40 and polyethylene glycol (40) monostearate.

17. The method according to any of claims 12 to 16, wherein the aforementioned surfactant is contained in about 0.01 (w/v)% - about 0.1 (w/v)%.

18. The method according to any of claims 12 to 16, wherein the aforementioned surfactant is contained in about 0.03 (w/v)% - about 0.1 (w/v)%.

19. The method according to any of claims 12 to 18, wherein the aforementioned resin is an optionally substituted polyolefin or a polyterephthalic acid ester.

20. The method according to any of claims 12 to 18, wherein the aforementioned resin is polyethylene, polypropylene, poly(vinylidene fluoride) or poly(ethylene terephthalate).
